(19) Europäisches Patentamt
European Patent Office
Office européen des brevets

(11) **EP 2 575 503 B1**

(12) **EUROPEAN PATENT SPECIFICATION**

(45) Date of publication and mention of the grant of the patent:
**30.04.2014  Bulletin 2014/18**

(21) Application number: **11716968.0**

(22) Date of filing: **05.05.2011**

(51) Int Cl.:
*A23L 1/22* (2006.01)  *A23F 3/24* (2006.01)
*A23F 3/14* (2006.01)  *A23F 3/00* (2006.01)
*A23F 3/34* (2006.01)  *A61K 36/48* (2006.01)
*A61K 36/82* (2006.01)  *A23L 1/30* (2006.01)
*A23L 2/52* (2006.01)

(86) International application number:
**PCT/EP2011/057224**

(87) International publication number:
**WO 2011/151127 (08.12.2011 Gazette 2011/49)**

(54) **PRODUCT COMPRISING CATECHINS**

PRODUKT MIT CATECHINEN

PRODUIT COMPRENANT DES CATECHINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **04.06.2010  EP 10164932**

(43) Date of publication of application:
**10.04.2013   Bulletin 2013/15**

(73) Proprietors:
• **Unilever PLC**
  **London,  EC4Y 0DY (GB)**
  Designated Contracting States:
  **CY GB IE MT**
• **Unilever NV**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
• **BLYTH, Marian, Jean**
  **Bedford, Bedfordshire MK44 1LQ (GB)**

• **JONES, Timothy, Graham**
  **Bedford, Bedfordshire MK44 1LQ (GB)**
• **WILKINSON, Annabel, Louise**
  **Bedford, Bedfordshire MK44 1LQ (GB)**

(74) Representative: **Askew, Sarah Elizabeth**
  **Unilever Patent Group**
  **Colworth House**
  **Sharnbrook**
  **Bedford MK44 1LQ (GB)**

(56) References cited:
  **WO-A1-2007/053929     WO-A1-2007/101349**
  **CA-A1- 2 465 481**

• **MARNEWICK J L ET AL: "Chemoprotective properties of rooibos (Aspalathus linearis), honeybush (Cyclopia intermedia) herbal and green and black (Camellia sinensis) teas against cancer promotion induced by fumonisin B1 in rat liver", FOOD AND CHEMICAL TOXICOLOGY, PERGAMON, GB LNKD- DOI:10.1016/J.FCT. 2008.11.004, vol. 47, no. 1, 1 January 2009 (2009-01-01), pages 220-229, XP025769237, ISSN: 0278-6915 [retrieved on 2008-11-07]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD OF THE INVENTION**

The present invention relates to products comprising catechins. In particular the invention relates to the use of solids from *Aspalathus linearis* for providing improved products comprising catechins.

**BACKGROUND OF THE INVENTION**

Tea has recently gained wide attention for its potentially beneficial effects on human health. Several epidemiological, clinical and experimental studies have established a positive correlation between tea consumption and certain health benefits. Taken regularly, tea has been reported to help improve vascular function, combat fatigue, reduce cholesterol levels and increase feelings of vitality.

The health benefits of tea are primarily attributed to its high polyphenol content. Catechins, which include epigallocatechin gallate (EGCG), epicatechin (EC), epicatechin gallate (ECG), and epigallocatechin (EGC), are the major polyphenolic compounds in green tea. A positive correlation between catechin consumption and improved cardiovascular health has been established, and their use as antiallergic agents and cerebral function activators has been proposed.

Although some of the health benefits of tea may be apparent at consumption rates as low as three cups per day (see, for example, U. Peters et al., "Does tea affect cardiovascular disease? A meta-analysis.", American Journal of Epidemiology, 2001, 154: pp.495-503), many individuals do not even achieve this modest consumption rate on a long term basis.

There have been several attempts to provide products with enhanced levels of catechins. Unfortunately, however, the incorporation of increased amounts of catechins can lead to products that have poor taste characteristics.

European patent EP 1 297 749 B1 (Kao Corporation) discloses a beverage with a concentrated or purified tea extract incorporated therein. The beverage, which contains catechins in combination with quinic acid at a predetermined ratio, is said to have an improved taste. More specifically, the beverage is said to be free from the unpleasant aftertaste which would otherwise remain after alleviation of the bitterness or astringency peculiar to catechins by a sweetener or the like. However, the beverages disclosed therein require the addition of quinic acid, which may itself bring unwanted taste to the beverage. Furthermore, the beverages are still unpleasantly bitter, especially for consumers used to tea-based beverages having relatively low levels of catechins, such as black tea.

Thus we have recognised that there is a need to provide a product which delivers the benefits of high levels of catechins, and which has improved taste, especially in respect of bitterness and/or astringency. We have found that the use of solids from *Aspalathus linearis* (rooibos) unexpectedly allows for the formulation of a product with excellent taste characteristics even when elevated levels of catechins are incorporated therein.

Documents WO 2007/101349 and WO 2007/053929 disclose compositions comprising catechins and rooibos.

**TESTS AND DEFINITIONS**

As used herein the term "comprising" encompasses the terms "consisting essentially of" and "consisting of". All percentages and ratios contained herein are calculated by weight unless otherwise indicated. It should be noted that in specifying any range of values or amount, any particular upper value or amount can be associated with any particular lower value or amount.

The disclosure of the invention as found herein is to be considered to cover all embodiments as found in the claims as being multiply dependent upon each other irrespective of the fact that claims may be found with multiple dependency or redundancy.

***Aspalathus linearis***

*Aspalathus linearis* (also known as rooibos or redbush) is a shrub-like leguminous bush native to the Western Cape region of South Africa, where it is extensively cultivated for commercial use as a herbal tea. As used herein the terms "solids from *Aspalathus linearis*" and "rooibos solids" are used interchangeably and refer to dry material extractable from the leaves and/or stems of the plant *Aspalathus linearis*. The term "rooibos extract" refers to an extract that comprises solids from *Aspalathus linearis*.

**Catechins**

The term "catechins" is used herein as a generic term for epicatechins, non-epicatechins, and mixtures thereof. The term "epicatechins" refers to epicatechin, epigallocatechin, epichatechin gallate, epigallocatechin gallate and mixtures thereof. The term "non-epicatechin" refers to catechin, gallocatechin, catechin gallate, gallocatechin gallate and mixtures thereof.

### Beverage

As used herein the term "beverage" refers to a substantially aqueous drinkable composition suitable for human consumption. Preferably the beverage comprises at least 85% water by weight of the beverage, more preferably at least 90% and most preferably from 95 to 99.9%.

As used herein the term "packaged" means that the beverage is contained within a sealed package. Non-limiting examples of suitable packages include bottles, cans, cartons, pouches and sachets.

### Tea-based beverage

As used herein the term "tea-based beverage" refers to a beverage comprising at least 0.01 % tea solids by weight of the beverage. Preferably the tea-based beverage comprises from 0.04 to 3% tea solids, more preferably from 0.06 to 2%, most preferably from 0.1 to 1%.

### Tea solids

As used herein the term "tea solids" refers to dry material extractable from the leaves of the plant *Camellia sinensis* var. *sinensis* and/or *Camellia sinensis* var. *assamica.* The leaves may have been subjected to a so-called "fermentation" step wherein they are oxidised by certain endogenous enzymes that are released during the early stages of "black tea" manufacture. This oxidation may even be supplemented by the action of exogenous enzymes such as oxidases, laccases and peroxidises. Alternatively the leaves may have been partially fermented ("oolong tea") or may have remained substantially unfermented ("green tea").

### Determination of titratable acidity

Titratable acidity is a measure of the total amount of free acid present in a sample. It is determined by titration using a standard solution of sodium hydroxide (NaOH) as described below.

Sodium Hydroxide neutralises free acid, giving an increase in pH. The amount of sodium hydroxide required to raise the sample pH to 8.3 is proportional to the amount of free acid in the sample. Free acid in samples should be measured in units of g/L citric acid (anhydrous).

*Apparatus*

10 mL pipette.
25 mL burette.
pH meter (compatible with Orion Ross Sure-Flow electrode 81-65, or similar).
0.1 mol/L sodium hydroxide solution.
4 g/L citric acid (anhydrous) standard.

*Preparation of equipment*

1. The pH meter and electrode should be set up as per the manufacturer's instructions.
2. The burette must be rinsed with, and then filled with 0.1 M NaOH.
3. The actual strength of the NaOH should be checked by titrating a sample of 4 g/L citric acid. This must be carried out daily, or when a new batch of NaOH is produced.

*Titration of sample*

1. Pipette 10 ml of sample into a suitable beaker.

2. Insert electrode.

3. Add sodium hydroxide, while continually stirring, until a stable reading of pH 8.3 is achieved.

*Calculation of results*

Titratable acidity is expressed in terms of citric acid equivalents [i.e. g/L citric acid (anhydrous)]. The titratable acidity of a sample can be calculated as follows:

$$Titratable\ Acidity = \frac{Titre\ Volume}{1000} \times \frac{NaOH\ Concentration}{Valency\ of\ Acid} \times \frac{1000}{Sample\ Volume} \times RMM\ of\ Acid$$

where:

| | |
|---|---|
| NaOH concentration | = 0.1 mol/L |
| RMM of citric acid (anhydrous) | = 192.12 g/mol |
| Valency of citric acid | = 3 |
| Sample volume | = 10 mL |

For a sample this is equivalent to:

$$Titratable\ Acidity = \frac{Titre\ Volume}{1000} \times \frac{0.1}{3} \times \frac{1000}{10} \times 192.12$$

where the titre volume is the volume of 0.1 M NaOH (in mL) required to raise the sample pH to 8.3, and the result is expressed as g/L citric acid (anhydrous).

For the measurement of carbonated samples, testing must be preceded by full degassing, as dissolved $CO_2$ gives erroneous results by forming carbonic acid. Vacuum de-gassing of still products is not required.

## SUMMARY OF THE INVENTION

In a first aspect, the present invention provides a product comprising solids from *Aspalathus linearis* and at least 50 mg catechins, characterised in that the ratio of solids from *Aspalathus linearis* to catechins is from 10:1 to 1:20.

We have found that the use of solids from *Aspalathus linearis* allows the formulation of products with excellent taste characteristics that enable consumers to meet the daily intake of catechins required to achieve health benefits. In particular, we have found that use of solids from Aspalathus linearis can be used to alleviate the bitterness and/or astringency of catechins.

In order to prepare a product which contains a high level of catechins without being unpleasantly bitter and/or astringent, we have found that it is necessary to control the relative concentration of catechins and solids from Aspalathus linearis. Therefore, in products according to the current invention the ratio of solids from Aspalathus linearis to catechins is from 10:1 to 1:20. Preferably the ratio of solids from Aspalathus linearis to catechins is from 5:1 to 1:10, more preferably from 2:1 to 1:5, most preferably from 1:1 to 1:4.

In another aspect, the present invention provides use of solids from Aspalathus linearis for reducing or eliminating bitterness and/or astringency of catechins.

In a further aspect, the present invention provides a method of manufacturing a product comprising solids from Aspalathus linearis and catechins in a ratio of from 10:1 to 1:20, wherein a substance comprising the catechins is combined with a substance comprising the solids from Aspalathus linearis. Preferably the substance comprising the catechins comprises tea solids, more preferably green tea solids.

## DETAILED DESCRIPTION

The product of the present invention may be a food or a beverage product, or a precursor for preparing such a product, Preferably the product is a beverage, more preferably it is a packaged tea-based beverage.

Products according to the present invention comprise solids from Aspalathus linearis and at least 50 mg catechins. The total catechin content of the product is preferably such that consumers can meet the daily intake of catechins required to achieve health benefits in one or two servings. Therefore, a serving comprises at least 50 mg catechins, preferably at least 100 mg, more preferably at least 150 mg and most preferably from 200 mg to 1000 mg.

Preferably the product comprises at least 0.1 % (1 mg/g) catechins by dry weight, more preferably at least 1 % (10 mg/g), still more preferably at least 3% (30 mg/g) and most preferably from 7% (70 mg/g) to 100% (1000 mg/g). It is preferred that the total dry weight of the product is less than 200 g, more preferably from 0.5 g to 100 g, still more preferably from 1 g to 50 g.

The mass of the product required to deliver suitable amounts of catechins to a consumer will depend on the format of the product and the catechin content of the product. For convenience of transport and handling it is preferred that the product has a total mass of 0.5 g to 2100 g, more preferably 1 g to 1100 g and most preferably 2 g to 550 g.

In products according to the present invention, the catechins may comprise epicatechins, non-epicatechins, or a mixture thereof. Natural sources of catechins (e.g. tea leaves, cocoa beans) comprise a mixture of epicatechins and non-epicatechins. Thus, it is preferred that the catechins comprise both epicatechins and non-epicatechins.

We have found that the use of solids from *Aspalathus linearis* in combination with catechins is well-suited to delivering a beverage that is not bitter. Thus in a preferred embodiment the product is a beverage, preferably a tea-based beverage, more preferably a green tea-based beverage. It is also envisaged that the product of the invention may be a beverage precursor.

In order to control the perception of sour taste, initial bitterness and/or bitterness aftertaste, it preferred that the titratable acidity of the product is from 0.1 to 3.0, more preferably from 0.3 to 2.0, still more preferably from 0.3 to 1.5 and most preferably from 0.5 to 1.3. The titratable acidity will depend on the format and composition of the product. In one embodiment, the titratable acidity of a beverage product may be controlled by varying the amount of citric acid and/or malic acid in the formulation.

From a standpoint of microbial stability and taste, it is preferred that the product has an acidic pH. In particular, the pH (at 20°C) may be from 2 to 7, more preferably from 2 to 5, most preferably from 2.5 to 4.

The product of the present invention may be manufactured by any suitable means. The product is preferably manufactured by a method wherein a substance comprising catechins is combined with a substance comprising solids from *Aspalathus linearis*. Preferably the substance comprising catechins comprises tea solids, more preferably green tea solids.

**EXAMPLES**

The present invention will now be illustrated by reference to the following non-limiting examples.

**Example 1**

Solids from *Aspalathus linearis* ("rooibos solids") were assessed for their ability to mask the bitterness of catechins. Products comprising catechins and different types of tea solids from *Camellia sinensis* (white tea, oolong tea, black tea) were also assessed. A high catechin tea powder (Sunphenon 90XLB, supplied by Taiyo Kagaku KK) was used to adjust the catechin content of each of the products to 645 mg/L.

Products containing equivalent amounts of catechins were qualitatively assessed on their bitterness and astringency by 6 untrained individuals in informal tasting sessions. The individuals did not know the composition of the products presented. Products comprising rooibos solids (385 mg/L) and catechins were perceived as being noticeably less bitter than products comprising catechins and tea solids.

**Example 2**

Five beverage products were prepared according to an established formulation comprising sweeteners, flavourings and preservatives. Green tea powder (Ceytea™ from Premium Exports Ceylon Ltd) was included in each beverage product to provide a uniform amount of catechins. Solids from *Aspalathus linearis* (provided by rooibos extract I) were present in products 3, 4 and 5 (see Table 1). Additionally, the titratable acidity of the products was controlled by varying the amounts of citric and malic acids contained therein.

TABLE 1

| Ingredient (g/L) | Product 1 | Product 2 | Product 3 | Product 4 | Product 5 |
|---|---|---|---|---|---|
| Green tea powder* | 2.2 | 2.2 | 2.2 | 2.2 | 2.2 |
| Rooibos extract I[+] | - | - | 1.0 | 0.5 | 0.2 |
| Citric Acid | 0.7 | 0.35 | 0.7 | 0.35 | 0.5 |

(continued)

| Ingredient (g/L) | Product 1 | Product 2 | Product 3 | Product 4 | Product 5 |
|---|---|---|---|---|---|
| Malic Acid | 0.3 | 0.15 | 0.3 | 0.15 | - |
| | | | | | |
| **Catechins (mg/L)‡** | 594 | 594 | 594 | 594 | 594 |
| **Rooibos solids (mg/L)†** | - | - | 350 | 175 | 70 |
| **Radio\*\*** | - | - | 1:1.7 | 1:3.4 | 1:8.5 |
| **Titratable acidity** | 1.3 | 0.8 | 1.3 | 0.8 | 0.8 |

*Ceytea™ from Premium Exports Ceylon Ltd (Sri Lanka).

⁺From Plantextrakt GmbH & Co. KG.

‡Ceytea™ specification: 270 mg/g catechins.

†Rooibos extract I specification: 35% solids from *Aspalathus linearis.*

\*\*Weight ratio of solids from *Aspalathus linearis* to catechins.

The beverage products were filled into 330 ml cans and sealed. The cans were then pasteurised at 70°C for 10 minutes in a batch pasteuriser.

The beverage products were evaluated by a taste panel comprising trained panellists. The results of the evaluations (trial A and trial B) are discussed below. Trial A and trial B were conducted separately on different dates.

In trial A, product 1 (a high catechin beverage product that is reported by consumers as having a perceptibly bitter taste) was compared with products 2, 3 and 4. Product 1 was judged to have the highest initial bitterness of all the products tasted in trial A. Product 4 was judged to have the lowest initial bitterness, and was significantly ($p < 0.05$) lower in initial bitterness than product 1.

Product 1 was judged to have the lowest sweetness of all the products tasted in trial A and products 2 and 4 were both significantly ($p < 0.05$) higher in sweetness than product 1. In agreement with this finding, product 1 was also ranked most sour, with products 2 and 4 both being judged to be significantly ($p < 0.05$) less sour than product 1.

Additionally, product 4 was judged to have the smoothest mouthfeel, while product 1 ranked lowest in smoothness. Products 3 and 4 were both significantly higher in smoothness than product 1 ($p < 0.05$).

In trial B, product 1 was compared with products 4 and 5. Product 1 was judged to have the highest initial bitterness of all the products tasted in trial B. Product 4 was judged to have the lowest initial bitterness, and product 5 was also directionally lower in initial bitterness than product 1.

Product 1 was judged to be the most sour of all the products tasted in trial B, with products 4 and 5 both being judged to be significantly ($p < 0.05$) less sour than product 1. In agreement with this finding, product 1 was also judged to have the lowest sweetness, with products 4 and 5 both directionally higher in sweetness than product 1. Additionally, product 4 was judged to have the smoothest mouthfeel, while product 1 ranked lowest in smoothness. Product 5 was directionally higher in smoothness than product 1.

## Example 3

Six beverage products were prepared according to an established formulation comprising sweeteners, flavourings and preservatives. Green tea powder (Ceytea™ from Premium Exports Ceylon Ltd) was included in each beverage product to provide a uniform amount of catechins. Solids from *Aspalathus linearis* were present in products 4, 6, 7 and 8 (see Tables 1 and 2). Additionally, the titratable acidity of the products was controlled by varying the amounts of citric and malic acids contained therein (see Tables 1 and 2).

The beverage products were filled into 330 ml cans and sealed. The cans were then pasteurised at 70°C for 10 minutes in a batch pasteuriser.

TABLE 2

| Ingredient (g/L) | Product 6 | Product 7 | Product 8 |
|---|---|---|---|
| Green tea powder* | 2.2 | 2.2 | 2.2 |
| Rooibos extract II⁺ | 0.486 | - | - |
| Rooibos extract III‡ | - | 0.175 | - |

(continued)

| Ingredient (g/L) | Product 6 | Product 7 | Product 8 |
|---|---|---|---|
| Rooibos extract IV[†] | - | - | 0.583 |
| Citric Acid | 0.35 | 0.35 | 0.35 |
| Malic Acid | 0.15 | 0.15 | 0.15 |
| | | | |
| **Catechins (mg/L)** | 594 | 594 | 594 |
| **Rooibos solids (mg/L)** | 175 | 175 | 175 |
| **Titratable acidity** | 0.8 | 0.8 | 0.8 |
| *Ceytea™ from Premium Exports Ceylon Ltd, Sri Lanka (270 mg/g catechins).<br>[+]From Plantextrakt GmbH & Co. KG (36% rooibos solids).<br>[‡]From Afriplex (Pty) Ltd. (100% rooibos solids).<br>[†]From Afriplex (Pty) Ltd. (30% rooibos solids). | | | |

Product 1 is a high catechin beverage product that is judged to have a perceptibly bitter taste. Products 4, 6, 7 and 8 (collectively referred to as "rooibos products") all contain 175 mg/L rooibos solids. As can be seen from Tables 1 and 2, the rooibos products utilise different rooibos extracts (I, II, III and IV) to provide the rooibos solids.

The beverage products were evaluated by a taste panel comprising trained panellists. The results of the evaluation (trial C) are discussed below.

Product 1 was judged to have the highest initial bitterness of all the products tasted in trial C. Product 4 was judged to have the lowest initial bitterness, and the rooibos products were all directionally lower in initial bitterness than product 1. Product 1 was also judged to be the most sour of all the products tasted in trial C. The rooibos products were all judged to be significantly ($p < 0.05$) less sour than product 1.

Additionally, the mouthfeel of product 1 was assessed as being significantly ($p < 0.05$) less smooth and less thick than the rooibos products. Product 4 was directionally smoother and thicker than the other rooibos products (i.e. products 6, 7 and 8).

The data from trial C indicate that the four rooibos products are not significantly different to each other in taste, flavour or mouthfeel. This implies that the presence of rooibos solids (regardless of their source) is important when it comes to formulating high catechin products with excellent taste characteristics.

## Claims

1. A product comprising solids from Aspalathus linearis and at least 50 mg catechins, **characterised in that** the ratio of solids from Aspalathus linearis to catechins is from 10:1 to 1:20.

2. A product as claimed in claim 1 wherein the product comprises at least 0.1% catechins by dry weight.

3. A product as claimed in any one of the preceding claims wherein the product is a beverage.

4. A product as claimed in daim 3 wherein the product is a packaged tea-based beverage.

5. A product as claimed in claim 3 or claim 4 wherein the product has a titratable acidity of from 0.1 to 3.0.

6. A product as claimed in any one of claims 3 to 5 wherein the product has a pH of from 2 to 7.

7. A product as claimed in any one of the preceding claims wherein the catechins comprise epicatechins, non-epicatechins or a mixture thereof.

8. A product as claimed in any one of the preceding claims wherein ratio of solids from Aspalathus linearis to catechins is from 5:1 to 1:10.

9. A product as claimed in any one of the preceding claims wherein the product has a titratable acidity of 0.3 to 2.0.

10. A product as claimed in any one of the preceding claims wherein the product has a total mass of 0.5 g to 2100 g.

11. Use of solids from Aspalathus linearis for reducing or eliminating bitterness and/or astringency of catechins.

12. A method of manufacturing a product comprising solids from Aspalathus linearis and catechins in a ratio of from 10:1 to 1:20, wherein a substance comprising the catechins is combined with a substance comprising the solids from Aspalathus linearis.

13. A method of manufacturing a product according to daim 12 wherein the substance comprising the catechins comprises tea solids.

14. A method of manufacturing a product according daim 12 or daim 13 wherein the product comprises at least 50 mg catechins.


**Patentansprüche**

1. Produkt, das Feststoffe von Aspalathus linearis und mindestens 50 mg Catechine aufweist, **dadurch gekennzeichnet, dass** das Verhältnis zwischen den Feststoffen von Aspalathus linearis und Catechinen 10:1 bis 1:20 beträgt.

2. Produkt nach Anspruch 1, wobei das Produkt mindestens 0,1 % Catechine aufweist, und zwar auf das Trockengewicht bezogen.

3. Produkt nach einem der vorstehenden Ansprüche, wobei das Produkt ein Getränk ist.

4. Produkt nach Anspruch 3, wobei das Produkt ein abgepacktes Getränk auf Teebasis ist.

5. Produkt nach Anspruch 3 oder 4, wobei das Produkt eine titrierbare Acidität von 0,1 bis 3,0 aufweist.

6. Produkt nach einem der Ansprüche 3 bis 5, wobei das Produkt einen pH-Wert von 2 bis 7 aufweist.

7. Produkt nach einem der vorstehenden Ansprüche, wobei die Catechine Epicatechine, Nicht-Epicatechine oder ein Gemisch davon aufweisen.

8. Produkt nach einem der vorstehenden Ansprüche, wobei das Verhältnis zwischen den Feststoffen von Aspalathus linearis und Catechinen 5:1 bis 1:10 beträgt.

9. Produkt nach einem der vorstehenden Ansprüche, wobei das Produkt eine titrierbare Acidität von 0,3 bis 2,0 aufweist.

10. Produkt nach einem der vorstehenden Ansprüche, wobei das Produkt eine Gesamtmasse von 0,5 bis 2100 g aufweist.

11. Verwendung von Feststoffen von Aspalathus linearis zur Verminderung oder Beseitigung der Bitterkeit und/oder Adstringens von Catechinen.

12. Verfahren zum Herstellen eines Produktes, das Feststoffe von Aspalathus linearis und Catechine in einem Verhältnis von 10:1 bis 1:20 aufweist, wobei eine die Catechine aufweisende Substanz mit einer Substanz kombiniert wird, die Feststoffe von Aspalathus linearis aufweist.

13. Verfahren zum Herstellen eines Produktes nach Anspruch 12,

wobei die Catechine aufweisende Substanz Teefeststoffe aufweist.

14. Verfahren zum Herstellen eines Produktes nach Anspruch 12 oder 13,
wobei das Produkt mindestens 50 mg Catechine aufweist.


**Revendications**

1. Produit comprenant des fractions solides provenant d'Aspalathus linearis et au moins 50 mg de catéchines, **caractérisé en ce que** le rapport des fractions solides d'Aspalathus linearis aux catéchines est de 10:1 à 1:20.

2. Produit selon la revendication 1 dans lequel le produit comprend au moins 0,1 % de catéchines en poids sec.

3. Produit selon l'une quelconque des revendications précédentes dans lequel le produit est une boisson.

4. Produit selon la revendication 3 dans lequel le produit est une boisson à base de thé sous emballage.

5. Produit selon la revendication 3 ou la revendication 4 dans lequel le produit a une acidité titrable de 0,1 à 3,0.

6. Produit selon l'une quelconque des revendications 3 à 5 dans lequel le produit a un pH de 2 à 7.

7. Produit selon l'une quelconque des revendications précédentes dans lequel les catéchines comprennent des épicatéchines, des non-épicatéchines ou un mélange de celles-ci.

8. Produit selon l'une quelconque des revendications précédentes dans lequel le rapport des fractions solides d'Aspalathus linearis aux catéchines est de 5:1 à 1:10.

9. Produit selon l'une quelconque des revendications précédentes dans lequel le produit a une acidité titrable de 0,3 à 2,0.

10. Produit selon l'une quelconque des revendications précédentes dans lequel le produit a un poids total de 0,5 à 2100 g.

11. Utilisation de fractions solides d'Aspalathus linearis pour réduire l'amertume et/ou l'astringence des catéchines.

12. Procédé de fabrication d'un produit comprenant des fractions solides provenant d'Aspalathus linearis et des catéchines dans un rapport de 10:1 à 1:20, dans lequel une substance comprenant les catéchines est combinée à une substance comprenant les fractions solides provenant d'Aspalathus linearis.

13. Procédé de fabrication d'un produit selon la revendication 12 dans lequel la substance comprenant les catéchines comprend des fractions solides de thé.

14. Procédé de fabrication d'un produit selon la revendication 12 ou la revendication 13 dans lequel le produit comprend au moins 50 mg de catéchines.

**EP 2 575 503 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1297749 B1
- WO 2007101349 A
- WO 2007053929 A

### Non-patent literature cited in the description

- **U. PETERS et al.** Does tea affect cardiovascular disease? A meta-analysis. *American Journal of Epidemiology,* 2001, vol. 154, 495-503